# EUROPEAN PATENT APPLICATION

(11) **EP 4 424 664 A1**
(43) Date of publication of application: **04.09.2024**
(21) Application number: 22884928.7
(22) Date of filing: 07.03.2022
(51) Int. Cl.: C07C 227/40, C07C 229/08, C07C 227/04, C07C 227/18

(54) **METHOD FOR PURIFYING PREGABALIN**

(30) Priority: 26.10.2021 CN 202111246329
(71) Applicant: Zhejiang Huahai Pharmaceutical Co., Ltd., Taizhou, Zhejiang 317024 (CN)
(72) Inventor: WANG, Yougui, Taizhou, Zhejiang 317000 (CN); CAI, Changqing, Taizhou, Zhejiang 317000 (CN); TAN, Jun, Taizhou, Zhejiang 317000 (CN); ZHU, Yuanxun, Taizhou, Zhejiang 317000 (CN); YAN, Fengfeng, Taizhou, Zhejiang 317000 (CN)
(74) Representative: Pfenning, Meinig & Partner mbB
(86) International application number: PCT/CN2022/079456
(87) International publication number: WO 2023/071011

(57) **Abstract**

The present invention relates to a method for purifying pregabalin, comprising the following steps: 1) heating a reaction solution, maintaining the temperature and removing impurities; and 2) adding an acid into the reaction solution in step 1) to adjust the pH value, crystallizing and centrifugally drying the solution to obtain high-purity pregabalin. The pregabalin prepared and obtained by employing the purification method of the present invention has a product purity that reaches 99.8% or above, a product yield of 90% or above, an impurity X and impurity Y content that is ≤0.05%, and has no detected impurity peak the relative retention time of which is 1.3.

## Description

### TECHNICAL FIELD OF THE INVENTION

The present invention relates to the field of pharmaceutical and chemical industry, in particularly to a method for purifying Pregabalin.

### BACKGROUND OF THE INVENTION

*Pregabalin* is a novel gamma-aminobutyric acid (GABA) receptor agonist capable of blocking voltage-dependent calcium channels and reducing release of neurotransmitters, and is clinically for the treatment of peripheral neuralgia as well as for adjuvant treatment of local partial epileptic seizures. A plurality of methods for preparing Pregabalin are disclosed in the prior art, such as WO2006121557A1 discloses a simple method for preparing Pregabalin, which is carried out by a Hoffmann rearrangement of the compound (R)-(-)-3-(carbamoylmethyl)-5-methylhexanoic acid using a large quantity of liquid bromine in the presence of bromine and alkali metal hydroxide.

CN101910111B disclosed a method wherein using sodium hypochlorite is used as a substitute for bromine at a temperature of 50 °C to 70 °C. Although such reaction conditions can reduce the impurity with a relative retention time of 1.3 mentioned in the patent, the reaction temperature mentioned in the above patent was too high, resulting in a low reaction yield. Meanwhile, the amount of the impurity with a relative retention time of 1.3 is up to 0.25% in some batches of Pregabalin prepared by the above patented method, and also up to 0.1% in a particularly preferable embodiment, i.e., there exists a large difference in terms of the level of this impurity between different batches.

CN106748850A discloses a method for preparing Pregabalin by reacting (R)-(-)-3-(carbamoylmethyl)-5-methylhexanoic acid with a sodium hypochlorite aqueous solution at a low temperature, adding a reducing agent to destroy the sodium hypochlorite, finally adding a catalyst, introducing hydrogen to carry out a reduction reaction, filtering, adjusting the mother liquor with acid, filtering again, and adding the resulting wet product to a mixture of isopropanol and water for re-refining to obtain Pregabalin. In this method, (R)-(-)-3-carbamoylmethyl-5-methylhexanoic acid reacted with an sodium hypochlorite aqueous solution at low temperature, wherein, the reaction under such conditions may be insufficient or has lower conversion rate, resulting in unsatisfactory product yield. Additionally, the method also introduced palladium/carbon catalyst for hydrogenation reduction, although the contents of impurities X and Y in Pregabalin can be effectively reduced, the introduction of palladium/carbon catalyst will high likely result in the excessive heavy metal in the product of Pregabalin, meanwhile a certain safety risk is present during the use of palladium/carbon.

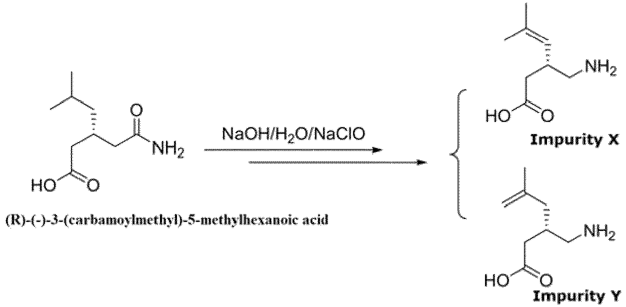

### SUMMARY OF THE INVENTION

The present invention provides a method for purifying Pregabalin, comprising the following steps:
(1) raising a temperature of a reaction solution, maintaining the temperature and removing impurities; and
(2) adding an acid into the reaction solution obtained in step (1) to adjust the pH value, crystallizing and centrifugally drying to obtain Pregabalin.

As a preferable embodiment, the reaction solution in step (1) is prepared by the following method:
(a) subjecting (R)-(-)-3-(carbamoylmethyl)-5-methylhexanoic acid and a sodium hypochlorite aqueous solution to an oxidation reaction in a sodium hydroxide aqueous solution, obtaining an oxidized reaction solution; and
(b) adding sodium sulfite to the oxidized reaction solution obtained in step (a), quenching the sodium hypochlorite, then obtaining the reaction solution.

As a preferable embodiment, the temperature in step (1) is 40 °C to 80 °C, preferably 55 °C to 70 °C.

As a preferable embodiment, the duration for maintaining the temperature in step (1) is 1 h to 5 h, preferably 2 h to 3 h.

As a preferable embodiment, the acid used in step (2) is hydrochloric acid, sulfuric acid or phosphoric acid, preferably hydrochloric acid.

As a preferable embodiment, the pH value in step (2) is adjusted to 5 to 9, preferably 6 to 8.

As a preferable embodiment, the mass concentration of the sodium hydroxide aqueous solution in step (a) is 10% to 40%, preferably 20% to 25%, and the mass concentration of the sodium hypochlorite aqueous solution is 5% to 14%, preferably 8 % to 12%.

As a preferable embodiment, the molar ratio of (R)-(-)-3-(carbamoylmethyl)-5-methylhexanoic acid, sodium hypochlorite and sodium hydroxide in step (a) is 1: (0.8 to 1.2): (2 to 5).

As a preferable embodiment, the sodium hypochlorite aqueous solution in step (a) is added dropwise to a mixed system of (R)-(-)-3-(carbamoylmethyl)-5-methylhexanoic acid and the sodium hydroxide aqueous solution.

As a preferable embodiment, the duration for the oxidation reaction in step (a) is 2 h to 20 h, preferably 5 h to 10 h, and the temperature for the oxidation reaction is -20 °C to 30 °C, preferably -10 °C to 30 °C.

As a preferable embodiment, the molar ratio of the added sodium sulfite to the (R)-(-)-3-(carbamoylmethyl)-5-methylhexanoic acid in step (b) is (0.01 to 0.50) : 1, preferably (0.02 to 0.15): 1.

The Pregabalin prepared by the method of the present invention has a product purity of 99.8% or more, a product yield of 90% or more, and a content of 0.05% or less for both impurity X and impurity Y, and has no detected impurity peak the relative retention time of which is 1.3.

Compared with the prior art, the present invention has the following beneficial effects:
(1) The preparation method of the present invention avoids the use of palladium/carbon and hydrogenation reduction operation, reduces the risk of excessive heavy metal residue in the product, simplifies the process operation, improves the production efficiency, shortens the production cycle, and thus is more conducive to large-scale industrialization.
(2) The preparation method of the present invention uses mild reaction conditions such that the whole process of the reaction is under control, avoiding side reactions caused by spontaneous exothermic heating up, and does not involve high temperature, high pressure, catalytic and other conditions, and the product has a low content of impurities, which is in line with the requirements of pharmaceutical applications.
(3) In the preparation method of the present invention, filtration can be carried out under the condition of cooling down to room temperature (20 °C to 30 °C) after the precipitation of the Pregabalin crystal by reasonably adjusting the dosage ratio of alkali and acid, without further cooling down to the condition of -10 °C to 10 °C for filtration, which saves a lot of energy and conforms to the requirements of energy conservation, emission reduction and green environmental protection.

### EXAMPLES

The following Examples are only for specific illustrations of the invention and are not intended to limit the scope of the invention.

### Example 1

66 g of 25% sodium hydroxide solution was added to the reaction flask, then was cooled down to a temperature of -20 °C. 20 g of (R)-(-)-3-(carbamoylmethyl)-5-methylhexanoic acid was added to the above solution, further, 72 g of 10.4% sodium hypochlorite solution was added slowly and dropwise. After the completion of the dropwise addition, the temperature was slowly raised to 10 °C, maintained for 3 h for reaction, and then raised to 30 °C for reaction for 3 h. 1.0 g of sodium sulfite was added to the reaction solution with stirring for 20 min, and the remaining amount of sodium hypochlorite was quenched, the reaction was ended. The reaction solution was heated up to 70 °C, maintained at this temperature for 2 h, and cooled down to 45 °C, further, 25 ml of hydrochloric acid was added dropwise to the reaction flask. The reaction solution was adjusted to a pH value of 7.0, cooled down to room temperature, filtered, and dried to obtain Pregabalin with a yield of 91.7%, a purity of 99.8%, a content of 0.04% for impurity X, and a content of 0.02% for impurity Y No impurity peak with relative retention time of 1.3 was detected.

### Example 2

830 g of 20% sodium hydroxide solution was added to the reaction flask, and then cooled down to a temperature of -15 °C. 200 g of (R)-(-)-3-(carbamoylmethyl)-5-methylhexanoic acid was added to the above solution, further, 720 g of 10.4% sodium hypochlorite solution was added slowly and dropwise. After the completion of the dropwise addition, the temperature was slowly raised to 10 °C, maintained for 4 h for reaction, and then raised to 30 °C for reaction for 3 h. 11.0 g of sodium sulfite was added to the reaction solution with stirring for 20 min, and the remaining amount of sodium hypochlorite was quenched, the reaction was ended. The reaction solution was heated up to 65 °C, maintained at this temperature for 2.5 h, and cooled down to 40 °C, further, 265 ml of hydrochloric acid was added dropwise to the reaction flask. The reaction solution was adjusted to a pH value of 7.1, cooled down to room temperature, filtered, and dried to obtain Pregabalin with a yield of 92.9%, a purity of 99.9%, a content of 0.03% for impurity X, and a content of 0.02% for impurity Y. No impurity peak with relative retention time of 1.3 was detected.

### Example 3

850 g of 22% sodium hydroxide solution was added to the reaction flask, and then cooled down to a temperature of -16 °C. 200 g of (R)-(-)-3-(carbamoylmethyl)-5-methylhexanoic acid was added to the above solution, further, 700 g of 10.6% sodium hypochlorite solution was added slowly and dropwise. After the completion of the dropwise addition, the temperature was slowly raised to 10 °C, maintained for 3 h for reaction, and then raised to 30 °C for reaction for 2 h. 10.0 g of sodium sulfite was added to the reaction solution with stirring for 20 min, and the remaining amount of sodium hypochlorite was quenched, the reaction was ended. The reaction solution was heated up to 60 °C, maintained at this temperature for 3 h, and cooled down to 50 °C, further, 280 ml of hydrochloric acid was added dropwise to the reaction flask. The reaction solution was adjusted to a pH value of 7.0, cooled down to room temperature, filtered, and dried to obtain Pregabalin with a yield of 92.3%, a purity of 99.8%, a content of 0.04% for impurity X, and a content of 0.02% for impurity Y. No impurity peak with relative retention time of 1.3 was detected.

### Example 4

420 kg of 20% sodium hydroxide solution was added to the reaction kettle, and then cooled down to -18 °C. 100 kg of (R)-(-)-3-(carbamoylmethyl)-5-methylhexanoic acid was added to the above solution, further, 350 kg of 10.4% sodium hypochlorite solution was added slowly and dropwise. After the completion of the dropwise addition, the temperature was slowly raised to 10 °C, maintained for 3 h for reaction, and then raised to 30 °C for reaction for 2 h. 14 kg of sodium sulfite was added to the reaction solution with stirring for 30 min, and the remaining amount of sodium hypochlorite was quenched, the reaction was ended. The reaction solution was heated up to 70 °C, maintained at this temperature for 3 h, and cooled down to 50 °C, further, 140 L of hydrochloric acid was added dropwise to the reaction flask. The reaction solution was adjusted to a pH value of 7.0, cooled down to room temperature, filtered, and dried to obtain Pregabalin with a yield of 93.7%, a purity of 99.9%, a content of 0.05% for impurity X, and a content of 0.02% for impurity Y. No impurity peak with relative retention time of 1.3 was detected.

## Claims

1. A method for purifying Pregabalin, comprising the following steps:
(1) raising the temperature of a reaction solution, maintaining the temperature and removing impurities; and
(2) adding an acid into the reaction solution obtained in step (1) to adjust the pH value, crystallizing and centrifugally drying to obtain Pregabalin.

2. The method for purifying Pregabalin according to claim 1, wherein the reaction solution in step (1) is prepared by the following steps:
(a) subjecting (R)-(-)-3-(carbamoylmethyl)-5-methylhexanoic acid and a sodium hypochlorite aqueous solution to an oxidation reaction in a sodium hydroxide aqueous solution, obtaining an oxidized reaction solution; and
(b) adding sodium sulfite to the oxidized reaction solution obtained in step (a), quenching the sodium hypochlorite, then obtaining the reaction solution.

3. The method for purifying Pregabalin according to claim 1, wherein the temperature in step (1) is 40 °C to 80 °C, preferably 55 °C to 70 °C.

4. The method for purifying Pregabalin according to claim 1, wherein the duration for maintaining the temperature in step (1) is 1 h to 5 h, preferably 2 h to 3 h.

5. The method for purifying Pregabalin according to claim 1, wherein the acid in step (2) is hydrochloric acid, sulfuric acid or phosphoric acid, preferably hydrochloric acid.

6. The method for purifying Pregabalin according to claim 1, wherein the pH value in step (2) is adjusted to 5 to 9, preferably 6 to 8.

7. The method for purifying Pregabalin according to claim 2, wherein the mass concentration of the sodium hydroxide aqueous solution in step (a) is 10% to 40%, preferably 20% to 25%, and the mass concentration of the sodium hypochlorite aqueous solution is 5% to 14%, preferably 8% to 12%.

8. The method for purifying Pregabalin according to claim 2, wherein the molar ratio of (R)-(-)-3-(carbamoylmethyl)-5-methylhexanoic acid, sodium hypochlorite and sodium hydroxide in step (a) is 1: (0.8 to 1.2) : (2 to 5).

9. The method for purifying Pregabalin according to claim 2, wherein the sodium hypochlorite aqueous solution in step (a) is added dropwise to a mixed system of (R)-(-)-3-(carbamoylmethyl)-5-methylhexanoic acid and the sodium hydroxide aqueous solution.

10. The method for purifying Pregabalin according to claim 2, wherein the duration for the oxidation reaction in step (a) is 2 h to 20 h, preferably 5 h to 10 h, and the temperature for the oxidation reaction is -20 °C to 30 °C, preferably -10 °C to 30 °C.

11. The method for purifying Pregabalin according to claim 2, wherein the molar ratio of the sodium sulfite to (R)-(-)-3-(carbamoylmethyl)-5-methylhexanoic acid in step (b) is (0.01 to 0.50) : 1, preferably (0.02 to 0.15) : 1.
